# EUROPEAN PATENT APPLICATION

(11) **EP 4 755 291 A1**
(43) Date of publication of application: **10.06.2026**
(21) Application number: 24849114.4
(22) Date of filing: 26.07.2024
(51) Int. Cl.: A61B 5/00, A45D 44/00, G01N 33/50, G06Q 50/10, G06T 7/00, G06V 10/70

(54) **INFORMATION PROCESSING SYSTEM**

(30) Priority: 28.07.2023 JP 2023123205; 12.01.2024 JP 2024003404
(71) Applicant: Kao Corporation, Tokyo 103-8210 (JP)
(72) Inventor: ICHIHASHI, Toshiki, Tokyo 103-8210 (JP); INOUE, Mayumi, Tokyo 103-8210 (JP); YANAGISAWA, Hiroki, Tokyo 103-8210 (JP); KIKUCHI, Sho, Tokyo 103-8210 (JP)
(74) Representative: Hoffmann Eitle
(86) International application number: PCT/JP2024/026892
(87) International publication number: WO 2025/028462

(57) **Abstract**

An information processing system comprises a control unit. The control unit generates a machine learning model by using training data, the training data being skin class information that is generated by analyzing biological information of multiple users provided by the multiple users and that classifies skins of the multiple users, the machine learning model estimating a skin class of the user from at least one of skeletal feature information or skin color information obtained by analyzing face images of the multiple users, the skeletal feature information indicating features of facial skeleton of the multiple users, the skin color information indicating features of skin colors of the multiple users. The control unit then receives an input of a face image of a user, analyzes the received face image to obtain at least one of the skeletal feature information or the skin color information, and outputs skin class information estimated from at least one of the obtained skeletal feature information or skin color information by using the machine learning model.

## Description

### Field of the Invention

The present invention relates to an information processing system, an information processing method, and a program for estimating a skin class of a user.

### Background of the Invention

Conventionally, there have been technologies of analyzing biological information of users and classifying the skins of the users. For example, Patent Literature 1 below describes that nucleic acids prepared from RNA collected from skin surface lipids of users are analyzed to classify the skins of the users into a group with a high amount of sebum and a group with a low amount of sebum, or into a group with a high content of skin moisture and a group with a low content of skin moisture.

### Citation List

### Patent Literature

Patent Literature 1: Japanese Patent Application Laid-open No. 2020-74769

### Summary of the Invention

### Technical Problem

However, in the technology of Patent Literature 1 described above, in order to classify the skins of the users, nucleic acids need to be prepared through a plurality of steps of converting the RNA collected from the skin surface lipids of the users into cDNA by reverse transcription and then performing multiplex PCR on the cDNA to purify the reaction product of the PCR, which requires time and effort.

A subject of the present invention relates to providing an information processing system, an information processing method, and a program that can more easily and quickly classify the properties of a skin of a user.

### Solution to Problem

An information processing system according to one embodiment of the present invention comprises a control unit. The control unit generates a machine learning model by using training data, the training data being skin class information that is generated by analyzing biological information of multiple users provided by the multiple users and that classifies skins of the multiple users, the machine learning model estimating a skin class of the user from at least one of skeletal feature information or skin color information obtained by analyzing face images of the multiple users, the skeletal feature information indicating features of facial skeleton of the multiple users, the skin color information indicating features of skin colors of the multiple users. The control unit then receives an input of a face image of a user, analyzes the received face image to obtain at least one of the skeletal feature information or the skin color information, and outputs skin class information estimated from at least one of the obtained skeletal feature information or skin color information by using the machine learning model.

An information processing method according to another embodiment of the present invention comprises: generating a machine learning model by using training data, the training data being skin class information that is generated by analyzing biological information of multiple users provided by the multiple users and that classifies skins of the multiple users, the machine learning model estimating a skin class of the user from at least one of skeletal feature information or skin color information obtained by analyzing face images of the multiple users, the skeletal feature information indicating features of facial skeleton of the multiple users, the skin color information indicating features of skin colors of the multiple users; receiving an input of a face image of a user; analyzing the received face image to obtain at least one of the skeletal feature information or the skin color information; and outputting skin class information estimated from at least one of the obtained skeletal feature information or skin color information by using the machine learning model.

A program according to still another embodiment of the present invention causes an information processing apparatus to execute the steps of: generating a machine learning model by using training data, the training data being skin class information that is generated by analyzing biological information of multiple users provided by the multiple users and that classifies skins of the multiple users, the machine learning model estimating a skin class of the user from at least one of skeletal feature information or skin color information obtained by analyzing face images of the multiple users, the skeletal feature information indicating features of facial skeleton of the multiple users, the skin color information indicating features of skin colors of the multiple users; receiving an input of a face image of a user; analyzing the received face image to obtain at least one of the skeletal feature information or the skin color information; and outputting skin class information estimated from at least one of the obtained skeletal feature information or skin color information by using the machine learning model.

### Advantageous Effects of Invention

The information processing system according to an embodiment of the present invention makes it possible to more easily and quickly classify the properties of a skin of a user.

### Brief Description of the Drawings

[Fig. 1] Fig. 1 is a diagram showing a configuration of a cosmetic information providing system according to one embodiment of the present invention.
[Fig. 2] Fig. 2 is a diagram showing a hardware configuration of a cosmetic information providing server according to one embodiment of the present invention.
[Fig. 3] Fig. 3 is a diagram showing a database configuration of the cosmetic information providing server according to one embodiment of the present invention.
[Fig. 4] Fig. 4 is a flowchart showing a flow of processing of generating a machine learning model for classifying a user's skin by the cosmetic information providing server according to one embodiment of the present invention.
[Fig. 5] Fig. 5 is a diagram showing skeletal feature information used to generate the machine learning model by the cosmetic information providing server according to one embodiment of the present invention.
[Fig. 6] Fig. 6 is a diagram showing skin color information used to generate the machine learning model by the cosmetic information providing server according to one embodiment of the present invention.
[Fig. 7] Fig. 7 is a flowchart showing a flow of user's skin class information and cosmetic information providing processing by the cosmetic information providing server according to one embodiment of the present invention.
[Fig. 8] Fig. 8 is a diagram showing a display example of cosmetic information provided by the cosmetic information providing server according to one embodiment of the present invention.
[Fig. 9] Fig. 9 is a diagram showing skin color information and eye color information used to generate the machine learning model by the cosmetic information providing server according to one embodiment of the present invention.

### Detailed Description of the Invention

Hereinafter, an embodiment of the present invention will be described with reference to the drawings.

### [Configuration of System]

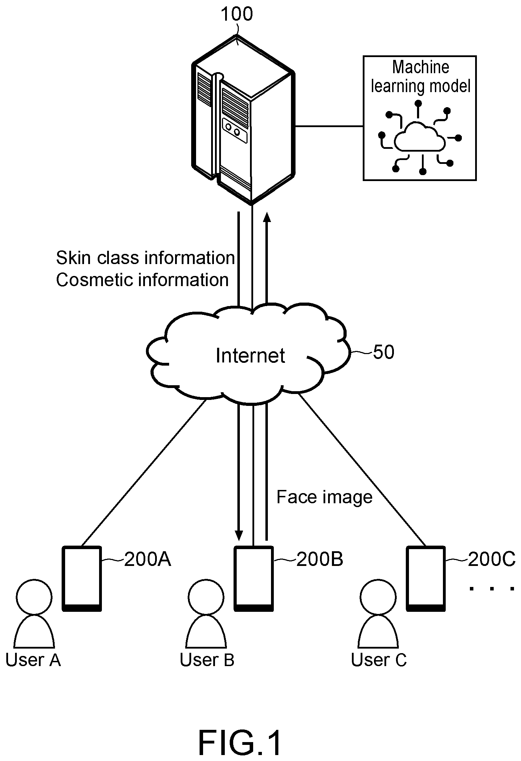

As shown in Fig. 1, this system includes a cosmetic information providing server 100 and a plurality of user terminals 200 on the Internet 50.

The cosmetic information providing server 100 is a web server (information processing apparatus) that is operated by an operator of a portal site (cosmetic information providing site) in which information related to cosmetics (including topical products such as quasi-drugs) can be searched for. The cosmetic information providing server 100 is connected to the plurality of user terminals 200 via the Internet 50.

The cosmetic information providing server 100 provides a cosmetic information providing service for the users of the user terminals 200 in the portal site. Specifically, the cosmetic information providing server 100 searches for cosmetic information that matches search conditions on the basis of a search request from the user terminal 200, generates a web page showing search results, and transmits the web page to the user terminal 200. Information such as the names, features, and usages of the cosmetics, as well as evaluation information (evaluation values, word of mouth, and reviews) for the cosmetics from the multiple users may be provided as the cosmetic information.

Additionally, the cosmetic information providing server 100 provides, in addition to the cosmetic information, skin class information that classifies the skin type on the basis of a face image of each user in response to a request of the user. In order to provide the skin class information, the cosmetic information providing server 100 generates and stores a machine learning model that estimates a skin class from feature information obtained by analyzing the face image of the user. Specifically, the machine learning model uses, as training data, each user's known skin class information generated by analyzing biological information (RNA) of multiple users provided by the multiple users, and estimates a skin class of a user on the basis of skeletal feature information indicating a feature of facial skeleton of the user and skin color information indicating a feature of a skin color of the user, which are obtained from the face image of the user. The processing of generating the machine learning model will be described later.

The user terminals 200 (200A, 200B, 200C ...) are terminals used by the users and are, for example, smartphones, mobile phones, tablet personal computers (PCs), notebook PCs, and desktop PCs. The user terminal 200 accesses the cosmetic information providing server 100, receives a web page showing the above cosmetic information and the like, and displays the web page on a screen using a browser or the like. Additionally, an application corresponding to the cosmetic information providing service may be installed on the user terminal 200, and the user terminal 200 may access the cosmetic information providing server 100 by the application and display the cosmetic information.

### [Hardware Configuration of Cosmetic Information Providing Server]

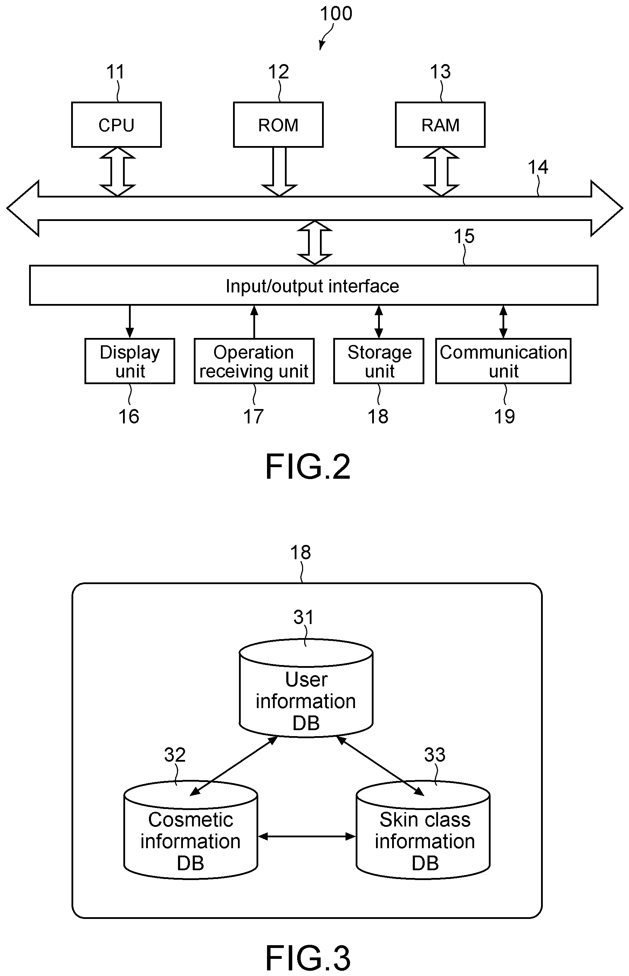

As shown in Fig. 2, the cosmetic information providing server 100 includes a central processing unit (CPU) 11, a read only memory (ROM) 12, a random access memory (RAM) 13, an input/output interface 15, and a bus 14 that connects the above components to each other.

The CPU 11 appropriately accesses the RAM 13 and the like as necessary and collectively controls the whole of each block of the cosmetic information providing server 100 while performing various types of arithmetic processing. A plurality of CPUs 11 may be provided depending on the processing. The ROM 12 is a non-volatile memory in which the operating system (OS) to be executed by the CPU 11 and firmware such as programs and various parameters are fixedly stored. The RAM 13 is used as a work area or the like for the CPU 11, and temporarily holds the OS, various applications being executed, and various types of data being processed.

A display unit 16, an operation receiving unit 17, a storage unit 18, a communication unit 19, and the like are connected to the input/output interface 15.

The display unit 16 is, for example, a display device using a liquid crystal display (LCD), an organic electroluminescence display (OELD), a cathode ray tube (CRT), or the like.

The operation receiving unit 17 is, for example, a pointing device such as a mouse, a keyboard, a touch panel, or another input device. If the operation receiving unit 17 is a touch panel, the touch panel may be integrated with the display unit 16.

The storage unit 18 is, for example, a non-volatile memory such as a hard disk drive (HDD), a flash memory (SSD; solid state drive), or another solid-state memory. The storage unit 18 stores the OS, various applications, and various types of data.

As will be described later, particularly in this embodiment, the storage unit 18 includes a user information database, a cosmetic information database, and a skin class information database, in addition to programs such as applications necessary for processing of generating a machine learning model for skin classifying processing, which will be described later, and the skin classifying processing using the machine learning model. The programs may be provided as program products.

The communication unit 19 is, for example, various modules for wireless communication, such as Ethernet NIC (Network Interface Card) or a wireless LAN, and performs processing of communicating with the user terminals 200.

Although not shown in the figures, the basic hardware configuration of the user terminal 200 is also substantially similar to the hardware configuration of the cosmetic information providing server 100.

### [Database Configuration of Cosmetic Information Providing Server]

As shown in Fig. 3, the cosmetic information providing server 100 includes a user information database 31, a cosmetic information database 32, and a skin class information database 33 in the storage unit 18. Note that each database may be stored not in the storage unit 18 but in a storage device or server externally connected to the cosmetic information providing server 100.

The user information database 31 stores, for each user, attribute information of a user of a cosmetic information providing service by the cosmetic information providing server 100. The attribute information of the user includes general information such as name, user ID for identifying the user, age (age group), occupation, address (area of residence), gender, and e-mail address, as well as information related to the user's dermis (skin), such as a skin type that the user perceives or understands, preferences of cosmetics, and skin problems that the user has. Note that the user ID functions as account information for using this cosmetic information providing service. The attribute information of the user is preferably selectable from multiple options. For example, in regard to a skin type, the attribute information of the user may be selected from multiple perspectives such as classification of dry skin or oily skin, a level of sensitive skin, a skin color, and the like.

The cosmetic information database 32 stores information related to cosmetics, that is, information such as manufacturer name, product name (brand name), and price of each cosmetic, category of cosmetic (e.g., serum, toner, cleanser, facial wash, makeup base, foundation, facial mask, cream, etc.), expected effects (e.g., brightening, pore refining treatment, acne skincare, exfoliating treatment, skin elasticity, sensitive skin care, moisture, anti-aging, luster, natural makeup, water proof, color retention, pigmentation, and the like).

Additionally, the cosmetic information database 32 also stores evaluation information of users with respect to cosmetics (multi-level evaluation value information and text information describing word of mouth). The evaluation information also includes data related to the time (month and day, or season) and location (region/area from which the user terminal 200 has access), at which the user provides the above evaluation information. The evaluation information is also stored in association with the user ID of the user who has input the evaluation information and the skin class information of the user.

The skin class information database 33 stores skin class information of each user, which has been generated using the machine learning model, in association with the user ID. The skin class information database 33 also stores known skin class information of each user generated by analyzing biological information of each user, which has been obtained as training data for generating the machine learning model, face image data of each user corresponding to the known skin class information, which has been used to generate the machine learning model, skeletal feature information and skin color information obtained by analyzing the face image data, other information such as parameters, and the like. As the face image data, a VISIA image captured by VISIA (registered trademark) that is a skin image analyzing apparatus or a smartphone image captured by the user using the user terminal 200 (smartphone) is used. The imaging direction is, for example, the front of the face, but images in other imaging directions in addition to or instead of the front of the face may also be used.

Those databases are referred to each other and used as necessary in the processing of generating a machine learning model and skin class information providing processing using the machine learning model by the cosmetic information providing server 100 to be described later.

### [Skin Class Information Derived from Biological Information]

Here, an example of a method of generating the skin class information by analyzing user's biological information will be described.

The operation company that generates the skin class information sends a biological sample kit to each user, and the user sends back a biological sample collected using the kit to the operator. The biological information is, for example, RNA information, and the biological sample kit is a tool for collecting a biological sample of each user, such as sebum, stratum corneum, saliva, urine, or blood. Sebum is preferable as a biological sample from the viewpoint of convenience in collecting RNA information and from the viewpoint of being able to identify a skin surface site.

Subsequently, the operation company measures RNA information from the biological sample collected from the user. Specifically, the RNA information is data obtained by extracting RNA from each biological sample for preparation, generating cDNA by reverse transcription, and then measuring the expression level for each of RNA species by sequencing, PCR, or the like. Known devices, means, and algorithms can be used to measure and analyze the RNA information from the biological sample in this case.

Subsequently, the operation company performs a cluster analysis of the RNA expression level data. Specifically, two or more skin physical property items are selected from multiple skin physical property items (e.g., stratum corneum water content, TEWL (transepidermal water loss), sebum content, melanin content, erythema level, overall redness, skin elasticity, etc.) for evaluating the skin physical properties of the user.

The degree of similarity is then determined on the basis of the expression levels (expression patterns) of the genes relevant to each sample related to the selected skin physical property items. A clustering method may be used as the method of determining the degree of similarity. Either hierarchical clustering or non-hierarchical clustering may be used as the clustering method. Other evaluation models such as a machine learning model may also be used.

Subsequently, a predetermined number of skin classes are generated corresponding to the number of clusters classified by the clustering. In this embodiment, for example, two skin classes (e.g., keratinizing type and immune type) are generated. Additionally, three types of skin classes may also be generated by adding a type in between both the above types.

Note that, as a result of the analysis, the following skin features were observed in the above keratinizing type and immune type.

### (Keratinizing Type)

- High melanin content
- Firmness
- Skin elasticity and no noticeable sagging around the corners of the mouth

### (Immune Type)

- Low melanin content, bright
- Less firmness
- Less skin elasticity and noticeable sagging around the corners of the mouth

The immune type also showed such lifestyle features as irregular meal times, instability around menstruation, and being susceptible to mental fatigue (stress).

Additionally, multiple types of the skin class may be generated by generating multiple types of different combinations of multiple skin physical property items. For example, RNA (genes) can be selected on the basis of the skin physical property items that are tailored to various skin problems such as spots and wrinkles. Further, in addition to RNA information, proteome information and information on skin surface bacteria may be combined to perform clustering.

Note that, in addition to the skin classes based on specific skin physical property items, skin classes based on RNA information may be provided by clustering expression data for between 1,000 and 20,000 genes. For example, clustering is performed on the basis of the expression levels of the genes relevant to a certain function (function X) and the genes relevant to another function (function Y), thus making it possible to generate four skin classes of a class I (function X: medium expression level; function Y: medium expression level), a class II (function X: medium expression level; function Y: low expression level), a class III (function X: high expression level; function Y: high expression level), and a class IV (function X: low expression level; function Y: medium expression level). In other words, if RNA profiles of users are close, it can be presumed that the users have similar skin problems or that the effects of cosmetics are also close, so that skin classes can be generated only by the degree of similarity in gene information (RNA expression level) without being associated with a specific skin physical property item.

### [Operation of Cosmetic Information Providing Server]

Next, the operation of the cosmetic information providing server 100 configured as described above will be described. The operation is performed by the cooperation of hardware such as the CPU 11, the communication unit 19, and the like of the cosmetic information providing server 100, and software stored in the storage unit 18. In the following description, for the purpose of convenience, the CPU 11 will be considered to be a main operating subject.

### (Processing of Generating Machine Learning Model)

First, the processing of generating a machine learning model for generating skin class information of a user by the cosmetic information providing server 100 will be described.

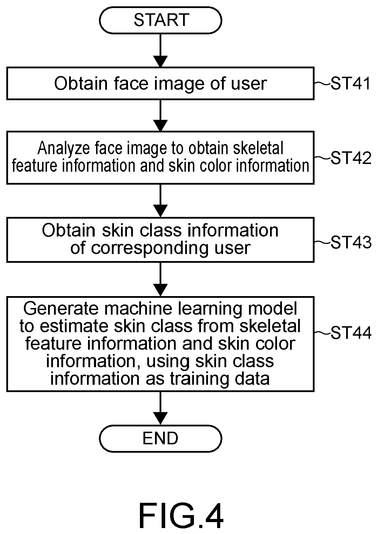

As shown in Fig. 4, the CPU 11 of the cosmetic information providing server 100 obtains a face image of a user from the skin class information database 33 (Step 41).

Note that, if a VISIA image is used as a face image for learning, the number of images is increased by, for example, rotating or inverting the original VISIA image. In this case, the number of increased images is adjusted such that the number of images for learning in each skin class is almost equally provided.

Additionally, if a smartphone image is used as a face image for learning, since parts other than the face may be shown in the captured image, pre-processing of extracting the face part from each face image may be performed, and further, pre-processing of cutting out only an image of a specific feature region from the face image may be performed. After those pre-processing, the processing of increasing the number of images is performed as in the case of the VISIA image.

Subsequently, the CPU 11 analyzes each face image to obtain skeletal feature information and skin color information as feature amounts (Step 42). The skeletal feature information is information indicating the feature of the user's facial skeleton (shape), and the skin color information is information indicating the feature of the user's skin color.

Here, the skeletal feature information is used, because it is assumed that, as described above, in the skin classes, the keratinizing type has the features of having firmness and elasticity and having less noticeable sagging around the corners of the mouth, whereas the immune type has the features of lack of firmness and elasticity and having noticeable sagging around the corners of the mouth, leading to a possibility that the keratinizing type and the immune type can be classified by analyzing the facial skeleton (facial shape) from the face image.

Additionally, the skin color information is used, because it is assumed that, as described above, in the skin classes, the keratinizing type has the features related to a skin color, such as having a high melanin content, whereas the immune type has the features related to a skin color, such as having a low melanin content and being bright, leading to a possibility that the keratinizing type and the immune type can be classified by analyzing the skin color from the face image.

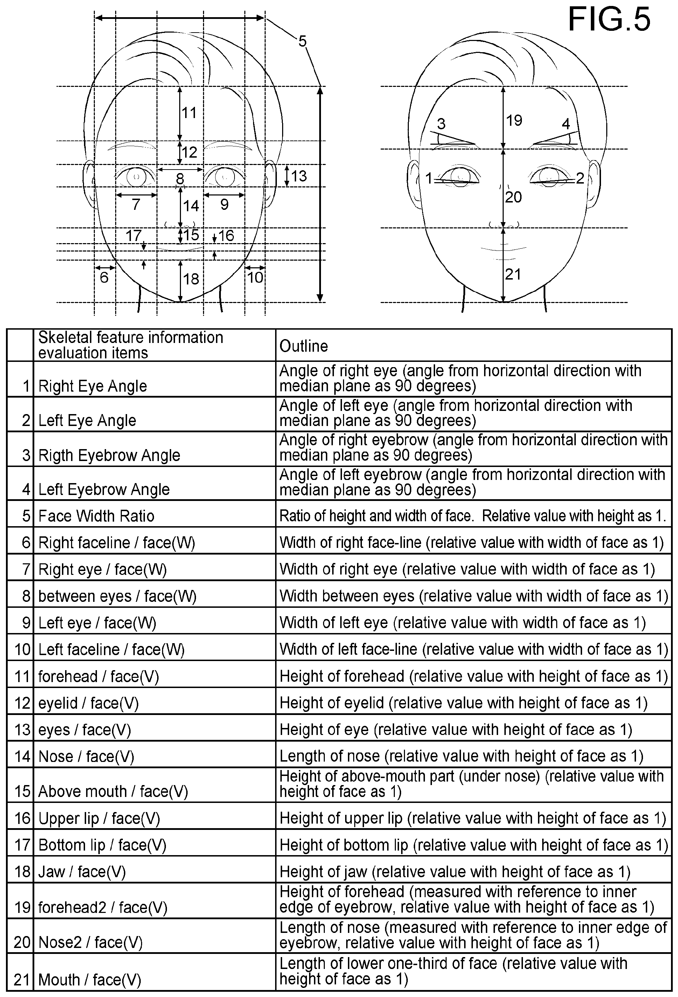

Specifically, as the skeletal feature information, length (ratio) information of each site of the face over a total of 21 items is obtained from the face image, for example, as shown in Fig. 5.

In other words, the following information is obtained from the face image: the angles of the left eye and the right eye (2 and 1 in the figure) and the angles of the left eyebrow and the right eyebrow (4 and 3 in the figure), a ratio of the height and the width of the face (5 in the figure), a ratio of each width of the left eye and the right eye to the width of the face (9 and 7 in the figure), a ratio of a distance between the left eye and the right eye (8 in the figure), each ratio of the width of the right face-line (lateral distance between the right temple and the right eye corner) and of the width of the left face-line (lateral distance between the left temple and the left eye corner) to the width of the face (6 and 10 in the figure), a ratio of the height of the forehead to the height of the face (11 in the figure), a ratio of each height of the left eye and the right eye to the height of the face (13 in the figure) and a ratio of each height of the left eyelid and the right eyelid to the height of the face (12 in the figure), a ratio of the height of the nose to the height of the face (14 in the figure), each ratio of the height of the upper lip, the height of the bottom lip, the height of the above-mouth part, and the height of the jaw to the height of the face (16, 17, 15, and 18 in the figure), and the like.

The skeletal feature information to be obtained is not limited to those shown in the figure, and not all of the 21 items mentioned above but some of those items may be obtained.

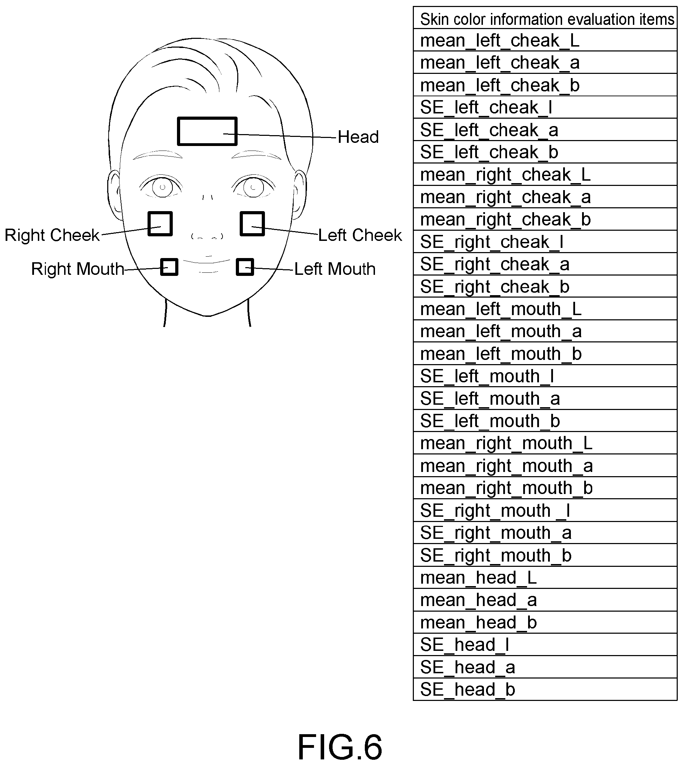

Additionally, as the skin color information, for example, as shown in Fig. 6, mean values (mean) of L*, a*, and b* (skin color) and standard error (SE) values of L*, a*, and b* (color unevenness) in the Lab color space are calculated for each of the forehead region, the left and right cheek regions, and the left and right corner-of-mouth regions in the face image.

In addition to or instead of them, median values and standard deviations (SD) of L*, a*, and b* in each region may be calculated. Additionally, color values in a color system other than the Lab color space may also be used. Further, the target regions are not limited to the five regions described above, but may be other regions as well, and the above-mentioned values may be calculated for some of the regions instead of all the five regions.

Subsequently, the CPU 11 obtains, from the skin class information database 33, known skin class information (e.g., keratinizing type, immune type) of the user, which has been associated with the face image from which the skeletal feature information and the skin color information have been obtained (Step 43).

The CPU 11 then generates a machine learning model that estimates a skin class from the obtained skeletal feature information and skin color information, using the known skin class information as training data (Step 44).

For example, a convolutional neural network (CNN), more specifically, VGG16 (CNN consisting of 16 layers), is used as a machine learning model, which is not limited to the above. For example, conventional machine learning models such as random forest, support vector machine, and logistic regression may be used.

The CPU 11 repeatedly performs the learning processing including adjustment of the skeletal feature information and skin color information and other parameters, until the estimation accuracy of the skin class by the above machine learning model is equal to or larger than a predetermined value.

### (Processing of Providing Skin Class Information and Cosmetic Information)

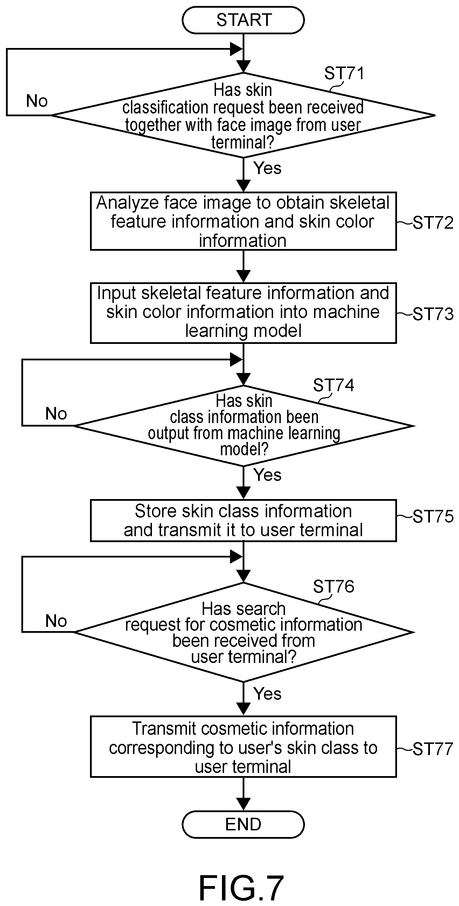

Next, processing of providing the skin class information and the cosmetic information to the user terminal 200 will be described. Fig. 7 is a flowchart showing a flow of the processing of providing the skin class information and the cosmetic information.

As shown in the figure, first, the CPU 11 determines whether a skin classification request has been received together with the face image from the user terminal 200 (Step 71). The skin classification request can be transmitted, for example, via a page for transmitting a skin classification request, which is provided on the cosmetic information providing site.

If it is determined that the skin classification request has been received (Yes in Step 71), the CPU 11 analyzes the received face image to obtain skeletal feature information and skin color information (Step 72).

Subsequently, the CPU 11 inputs the skeletal feature information and skin color information obtained from the face image into the machine learning model (Step 73).

Subsequently, the CPU 11 determines whether or not the skin class information has been output from the machine learning model (Step 74). If it is determined that the skin class information has not been output (No in Step 74), the CPU 11 repeats the confirmation until the skin class information is output.

If it is determined that the skin class information has been output from the machine learning model (Yes in Step 74), the CPU 11 associates this skin class information with a user ID of the user as a request source to store it in the skin class information database 33, and also transmits the skin class information to the user terminal 200 as the request source (Step 75).

Subsequently, the CPU 11 determines whether or not a search request for cosmetic information has been received from the user terminal 200 to which the skin class information has been transmitted (Step 76).

If it is determined that a search request for cosmetic information has been received (Yes in Step 76), the CPU 11 transmits a cosmetic information providing page, showing search results of the cosmetic information corresponding to the skin class associated with the user ID of the user who has transmitted the search request, to the user terminal and causes the user terminal to display the cosmetic information providing page (Step 77).

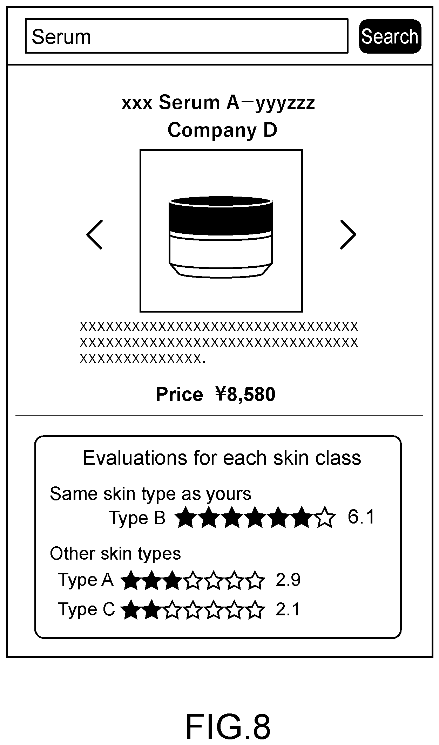

Fig. 8 is a diagram showing a display example of the cosmetic information providing page.

As shown in the figure, the cosmetic information providing page shows, for example, as information indicating search results of a serum, evaluation information (e.g., 7-level evaluation) on the product by users with the same skin class (e.g., skin type B) as that of the user who has transmitted the search request, and evaluation information on the product by users with other skin classes (e.g., skin types A and C)), together with information introducing the serum (product name, photo, description, price, etc.). This allows the user to easily find a product that matches the user's skin class and has a high evaluation.

Note that only the evaluation information of the skin class of the user who has transmitted the search request (type B in the figure) may be extracted and published without publishing the evaluation information of the skin classes (types A and C in the figure), which are not the skin class of the user who has transmitted the search request.

As described above, according to this embodiment, the cosmetic information providing server 100 can more easily and quickly classify the properties of the user's skin and provide cosmetic information corresponding thereto by simply obtaining the user's face image, without having to take the trouble to collect and analyze biological information (RNA) from the user to generate skin class information.

### [Modified Examples]

Hereinabove, the embodiment of the present invention has been described, but the present invention is not limited to the embodiment described above and can be variously modified without departing from the gist of the present invention as a matter of course.

In the embodiment described above, the cosmetic information providing server 100 generates a machine learning model on the basis of the skeletal feature information and skin color information obtained from the face image, but a machine learning model that estimates a skin class using only one of the skeletal feature information and the skin color information may be generated. Further, in addition to or instead of the skeletal feature information and the skin color information, skin surface information (information indicating the states of desquamation, psoriasis, wrinkles, texture, pores, and the like) may also be taken into account as parameters to generate a machine learning model that estimates a skin class.

Additionally, in the embodiment described above, the cosmetic information providing server 100 may train a machine learning model so as to estimate a skin class using eye color information in addition to the skeletal feature information and/or the skin color information obtained from the face image. Further, in this case, some of the evaluation items shown in Figs. 5 and 6 may not be calculated as the skeletal feature information and/or the skin color information.

The cosmetic information providing server 100 then analyzes the received face image to obtain at least one of the skeletal feature information and/or the skin color information and the eye color information, and outputs skin class information estimated from the obtained at least one of the skeletal feature information or the skin color information and the eye color information, by using the machine learning model.

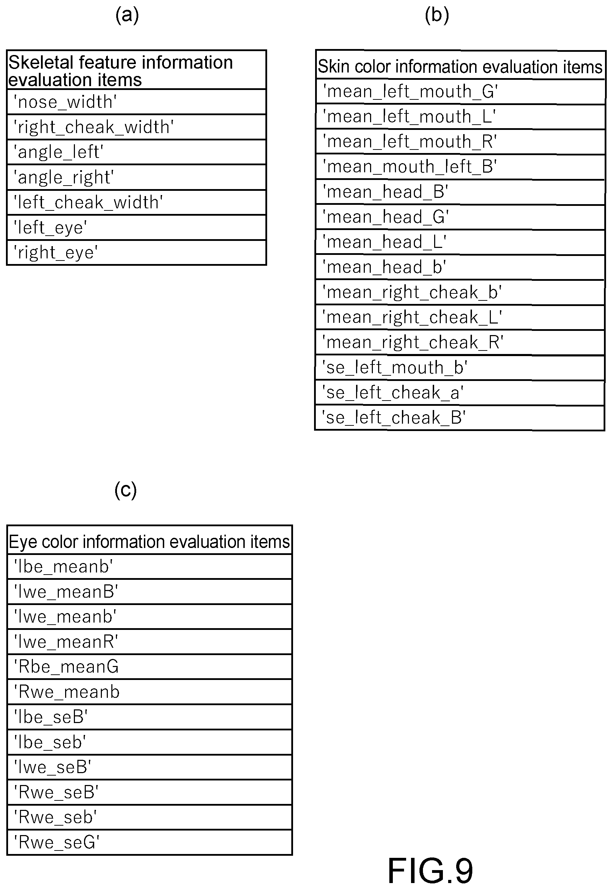

For example, as shown in (b) and (c) of Fig. 9, the cosmetic information providing server 100 calculates, as skin color information, the mean values (mean) of R, G, and B in the RGB, the mean values (mean) of L*, a*, and b* in the Lab color space, and the standard error (SE) values of R, G, and B and L*, a*, and b* (color unevenness) for each of the forehead region, the left and right cheek regions, and the left and right corner-of-mouth regions in the face image, and also calculates, as information indicating the color of eye (eye color information), the mean values and SE values of R, G, and B and L*, a*, and b* in the iris (pupil) regions of the left and right eyes and the surrounding white regions.

In this case, specifically, as shown in (a) of Fig. 9, the width of the nose, the width of each of the left and right cheeks (each distance from the left/right edge of the nose to the left/right edge of the face), and the angles and widths of the left and right eyes may be selectively calculated as the skeletal feature information.

Additionally, as shown in (b) of Fig. 9, the mean values of R, G, B, and L* in the left corner-of-mouth region, of G, B, L*, and b* in the forehead region, and of R, L*, and b* in the right cheek region, and the SE values of b* in the left corner-of-mouth region, and of B and a* in the left cheek region may be selectively calculated as the skin color information.

In addition, as shown in (c) of Fig. 9, the mean values of b* in the left-eye iris region, of R, B, and b* in the left-eye white region, of G in the right-eye iris region, and of b* in the right-eye white region, and the SE values of B and b* in the left-eye iris region, of B in the left-eye white region, and of G, B, and b* in the right-eye white region may be selectively calculated as the eye color information.

As a matter of course, the evaluation items to be calculated are not limited to those above, and evaluation items for different regions may also be calculated. For example, as eye color information, only the color of one of the left and right eyes may be used, or only the color of the iris region or the white region of both or one of the eyes may be used. Additionally, in this modified example using the eye color information as well, in addition to or instead of the above, median values or standard deviations (SD) of R, G, and B or L*, a*, and b* in each region may be calculated, or color values in a color system other than the RGB and the Lab color space may be used.

In such a manner, the estimation accuracy of the skin class can be improved by supplementarily using the eye color information (color, color unevenness) in addition to the above skeletal feature information and/or skin color information.

As a result of analyzing, by the inventors of the present invention, the user's skin image used to obtain the skin color information and the skeletal feature information in the embodiment described above, the inventors have found that, as a prediction confidence (a statistical measure indicating how certain the prediction or output is) at the time of classifying a skin class using the machine learning model becomes higher, the classification accuracy (accuracy rate) becomes higher.

In particular, when the mean values of image feature amounts are compared between an image with high confidence and an image with low confidence, it has been found that the image with high confidence (e.g., 70% or higher) has a higher B value in RGB in the forehead, mouth, and eye regions than that of the image with low confidence (e.g., 50 to 53%), and the image with low confidence has a higher yellow tinge (b* value in Lab) in the forehead, cheek, and eye regions than that of the image with high confidence.

In this regard, the cosmetic information providing server 100 may receive multiple face images from the user, and extract, for analysis by the machine learning model, a face image with a higher mean value of the B values in the RGB values of the respective pixels of the face image among the multiple face images, or exclude, from the above analysis, a face image with a higher mean value of the b values in the Lab values of the respective pixels.

Specifically, the cosmetic information providing server 100 may extract a face image with the highest mean value of the B values in the RGB values from the multiple face images, or may receive again a face image from the user until a face image whose mean value of the B values in the RGB values is a predetermined threshold or more can be obtained. In this case, if the mean value of the B values in the RGB values of the obtained face image is less than a threshold, the cosmetic information providing server 100 may transmit message information such as "Please take a face image again." to the user terminal 200 and cause the user terminal 200 to output the message information so as to encourage the user to take another image.

Additionally, if the mean value of the b values in the Lab values of the obtained face image is a predetermined threshold or more, the cosmetic information providing server 100 may receive a face image again from the user in the same manner as described above until a face image whose mean value of the b values is less than a predetermined threshold can be obtained.

The cosmetic information providing server 100 may perform the calculation of the mean value of the B values in the RGB values exclusively for at least one of the forehead, mouth, or eye region, or may perform the calculation for the entire face image regardless of those regions. Similarly, the cosmetic information providing server 100 may perform the calculation of the mean value of the b values in the Lab values exclusively for the forehead, cheek, and eye regions, or may perform the calculation for the entire face image regardless of those regions.

Additionally, as a result of the above analysis of the skin image, the inventors of the present invention have found that the image with high confidence shows skin unevenness more clearly and has more fine shadows such as spots.

In this regard, the cosmetic information providing server 100 may receive multiple face images from the user, and extract, for the analysis by the machine learning model, a face image in which at least one of a spot, pores, a wrinkle, desquamation, psoriasis, or skin unevenness can be recognized, out of the multiple face images.

Specifically, when receiving a face image from the user, the cosmetic information providing server 100 performs image recognition processing to determine whether or not a spot, pores, a wrinkle, desquamation, psoriasis, or skin unevenness can be recognized, and if not recognized, may transmit message information such as "Please take a face image again, because the resolution is low." or "Please take a face image again in a brighter location." to the user terminal 200 and cause the user terminal 200 to output the message information so as to encourage the user to take another image until a recognizable face image can be obtained.

Additionally, the cosmetic information providing server 100 may extract, for the analysis, a face image in which edges can be detected by a predetermined edge detection filter generated on the basis of a face image with a sufficiently high resolution and high confidence, instead of the detection of spots, pores, and the like.

In the embodiment described above, an example has been described, in which a user who has transmitted a face image to the cosmetic information providing server 100 and has been notified of user's skin class information accesses a cosmetic search page to search for cosmetic information corresponding to the skin class. However, it is thought that there are users who access the search page without knowing information related to a skin class. In this regard, if a user who has not been notified of skin class information (a non-logged-in user or a logged-in user whose user ID has not been given a skin class) accesses a search page, the cosmetic information providing server 100 may display proposition information for proposing the user to provide skin class information using a face image (to transmit a face image for skin class), for example, in a pop-up form on the search page.

In the embodiment described above, RNA information is used as the biological information, but in addition thereto, proteomes (proteins) collected from the user's stratum corneum, sebum, and the like may be analyzed, or skin surface bacteria collected from the surface of the skin may be analyzed.

In the embodiment described above, cosmetics (including topical products such as quasi-drugs) have been described as examples of products. However, the products are not limited to cosmetics, and information related to any products that the user uses (also ingests) for the body (skin, hair, etc.), including, for example, hair care products such as shampoos, conditioners, and treatments, pharmaceuticals (such as skin pharmaceutical preparations), sweat wipes, cosmetic sheets, toothpaste, beverages, foods, facial equipment, beauty tools, and other products, may be provided according to the skin class information generated on the basis of the face image of the user.

In the embodiment described above, the single cosmetic information providing server 100 has been described, but the processing executed by the cosmetic information providing server 100 may be distributed and executed by multiple servers. For example, the processing of generating a machine learning model shown in Fig. 4 and the processing of providing skin class information and cosmetic information using the machine learning model shown in Fig. 7 may be executed by separate servers.

Additionally, in the embodiment described above, the web page created by the cosmetic information providing server 100 is displayed by a browser of the user terminal 200, but if an application program corresponding to the cosmetic information providing service is installed on the user terminal 200, the web page may be displayed by the application program.

In the embodiment described above, the present invention has been described as a cosmetic information providing system that outputs to the user terminal 200 cosmetic information corresponding to skin class information estimated by analyzing a face image using a machine learning model. However, the present invention may be achieved as an information processing system that simply outputs estimated skin class information without providing cosmetic information corresponding to the skin class information.

In the embodiment described above, the cosmetic information providing server 100 may obtain the skin color information by using an estimation model that has been trained to output a skin state of a person's face in a face image from the face image.

In other words, the cosmetic information providing server 100 may convert the face image such that multiple points on a face-line of a face region of a person's face in the obtained face image of the user are located at an image outer edge and obtain a converted face image in order to fit the input rules of the estimation model, input the converted face image into the estimation model, and obtain skin state information of the face of the user from the estimation model as the skin color information.

Here, the cosmetic information providing server 100 may exclude or reduce, in the converted face image, a predetermined local site region within the face region of the person's face appearing in the obtained face image. In other words, in the conversion processing, the cosmetic information providing server 100 may match, among multiple outer circumferential points surrounding the predetermined local site region within the face region, the positions of the outer circumferential points that mutually face each other through the predetermined local site region, and may exclude or reduce the above predetermined local site region within the face region. The predetermined local site region is, for example, an eye region, an eyebrow region, and a lip region.

Further, the cosmetic information providing server 100 may further execute position-identifying processing of identifying position coordinates of predetermined multiple locations in the face region of the person's face appearing in the obtained face image. In this case, in the conversion processing, the obtained face image may be converted by converting some or all of the identified position coordinates of the predetermined multiple locations so as to fit the input rules of the estimation model.

In this case, the face image may have a predetermined rectangular shape, and the position coordinates of the predetermined multiple locations may further include position coordinates of multiple points on the face-line in the face region, the tip of the nose in the face region, and other multiple points in the face region. In the conversion processing, the cosmetic information providing server 100 may perform coordinate conversion such that four first face-line points included in the multiple points on the face-line in the face region are disposed respectively at the apices of the predetermined rectangular shape, the tip of the nose is disposed at the center of the predetermined rectangular shape, the other points on the face-line in the face region are disposed on the sides of the predetermined rectangular shape, and the other multiple points in the face region move in accordance with the movement of the multiple points on the face-line in the face region and the tip of the nose.

Here, the other multiple points in the face region may include four third face-line points located closer to the face-line in the face region than the predetermined local site region, and four second face-line points located much closer to the face-line in the face region than the four third face-line points. The cosmetic information providing server 100 then may perform, in the conversion processing, coordinate conversion such that the four second face-line points and the four third face-line points are disposed on the diagonal lines respectively connecting the four first face-line points and the tip of the nose, and a difference between a distance from the tip of the nose to the first face-line point and a distance from the tip of the nose to the second face-line point is smaller than a difference between a distance from the tip of the nose to the second face-line point and a distance from the tip of the nose to the third face-line point.

In the inventions described in the scope of claims of the present application, the invention described as an "information processing method" is one in which at least one apparatus such as a computer automatically performs each step by information processing performed by software, and is not performed by a human using an apparatus such as a computer. In other words, the "information processing method" is an information processing method performed by computer software and is not a method by a human operating a computational tool called a computer.

## Claims

1. An information processing system, comprising:
a control unit that
generates a machine learning model by using training data, the training data being skin class information that is generated by analyzing biological information of multiple users provided by the multiple users and that classifies skins of the multiple users, the machine learning model estimating a skin class of the user from at least one of skeletal feature information or skin color information obtained by analyzing face images of the multiple users, the skeletal feature information indicating features of facial skeleton of the multiple users, the skin color information indicating features of skin colors of the multiple users,
receives an input of a face image of a user,
analyzes the received face image to obtain at least one of the skeletal feature information or the skin color information, and
outputs skin class information estimated from at least one of the obtained skeletal feature information or skin color information by using the machine learning model.

2. The information processing system according to claim 1, wherein
the control unit
analyzes the face image to obtain the skeletal feature information, and
outputs the skin class information estimated from the skeletal feature information by using the machine learning model.

3. The information processing system according to claim 1, wherein
the control unit generates the machine learning model by using the skin class information generated by analyzing RNA of the users as the training data.

4. The information processing system according to claim 1, wherein
the control unit
generates the machine learning model from the skeletal feature information and the skin color information,
analyzes the received face image to obtain the skeletal feature information and the skin color information, and
outputs skin class information estimated from the obtained skeletal feature information and skin color information by using the machine learning model.

5. The information processing system according to claim 1 or 2, wherein
the control unit uses, as the skeletal feature information, a ratio of a height and a width of a face in the face image.

6. The information processing system according to claim 1 or 2, wherein
the control unit uses, as the skeletal feature information,
a ratio of a width of a left eye and a ratio of a width of right eye to a width of a face in the face image, or
a ratio of a distance between a left eye and a right eye to a width of a face in the face image.

7. The information processing system according to claim 1 or 2, wherein
the control unit uses, as the skeletal feature information,
a ratio of a height of a left eye and a ratio of a height of a right eye to a height of a face in the face image, or
a ratio of a height of a left eyelid and a ratio of a height of a right eyelid to a height of a face in the face image.

8. The information processing system according to claim 1 or 2, wherein
the control unit uses, as the skeletal feature information,
an angle of a left eye and an angle of a right eye in the face image, and
an angle of a left eyebrow and an angle of a right eyebrow.

9. The information processing system according to claim 1 or 2, wherein
the control unit uses, as the skeletal feature information,
a ratio of a height of an upper lip, a ratio of a height of a bottom lip, and a ratio of a height of an above-mouth part to a height of a face in the face image.

10. The information processing system according to any one of claims 1 to 7, wherein
the control unit uses, as the skeletal feature information,
a ratio of a height of a nose or a ratio of a height of a forehead to a height of a face in the face image.

11. The information processing system according to claim 1 or 2, wherein
the control unit uses, as the skeletal feature information,
a ratio of a lateral distance between a right temple and a right eye corner to a width of a face in the face image, and
a ratio of a lateral distance between a left temple and a left eye corner to a width of a face in the face image.

12. The information processing system according to claim 1, wherein
the control unit uses, as the skin color information, at least one value of a mean value, a median value, a standard deviation, or a standard error of a color value of at least one region in the face image.

13. The information processing system according to claim 10, wherein
the control unit uses, as the skin color information, the at least one value of at least two regions of a forehead region, left-and-right cheek regions, and left-and-right mouth corner regions in the face image.

14. The information processing system according to claim 1 or 2, wherein
the control unit
receives, from a user terminal of the user, an input of a face image captured by the user terminal, and
outputs, to the user terminal, information about cosmetics corresponding to the skin class information estimated by analysis of the face image.

15. The information processing system according to claim 1, wherein
the control unit
trains the machine learning model such that the machine learning model estimates the skin class on a basis of at least one of the skeletal feature information or the skin color information obtained by analysis of a face image of a user and on a basis of eye color information indicating a feature of a color of an eye of the user,
analyzes the received face image to obtain at least one of the skeletal feature information or the skin color information and the eye color information, and
outputs skin class information estimated from the obtained at least one of skeletal feature information or skin color information and the eye color information by using the machine learning model.

16. The information processing system according to claim 1 or 2, wherein
the control unit
receives multiple face images from the user, and
extracts, for the analysis, a face image having a high mean value of B values of RGB values of pixels of the face image, out of the multiple face images, or
excludes, from the analysis, a face image having a high mean value of b values of Lab values of the pixels.

17. The information processing system according to claim 1 or 2, wherein
the control unit
receives multiple face images from the user, and
extracts, for the analysis, a face image in which a spot, pores, a wrinkle, desquamation, psoriasis, or skin unevenness is recognized, out of the multiple face images.

18. The information processing system according to claim 1, wherein
the control unit
converts the face image such that multiple points on a face-line of a face region of a person's face in the received face image are near an image outer edge, and obtains a converted face image,
inputs the converted face image into an estimation model, the estimation model being trained to output a skin state of a person's face in a face image from the face image, and
obtains skin state information of a face of the user from the estimation model as the skin color information.

19. An information processing method, comprising:
generating a machine learning model by using training data, the training data being skin class information that is generated by analyzing biological information of multiple users provided by the multiple users and that classifies skins of the multiple users, the machine learning model estimating a skin class of the user from at least one of skeletal feature information or skin color information obtained by analyzing face images of the multiple users, the skeletal feature information indicating features of facial skeleton of the multiple users, the skin color information indicating features of skin colors of the multiple users;
receiving an input of a face image of a user;
analyzing the received face image to obtain at least one of the skeletal feature information or the skin color information; and
outputting skin class information estimated from at least one of the obtained skeletal feature information or skin color information by using the machine learning model.

20. A program, that causes an information processing apparatus to execute the steps of:
generating a machine learning model by using training data, the training data being skin class information that is generated by analyzing biological information of multiple users provided by the multiple users and that classifies skins of the multiple users, the machine learning model estimating a skin class of the user from at least one of skeletal feature information or skin color information obtained by analyzing face images of the multiple users, the skeletal feature information indicating features of facial skeleton of the multiple users, the skin color information indicating features of skin colors of the multiple users;
receiving an input of a face image of a user;
analyzing the received face image to obtain at least one of the skeletal feature information or the skin color information; and
outputting skin class information estimated from at least one of the obtained skeletal feature information or skin color information by using the machine learning model.

21. A recording medium, that stores a program that causes an information processing apparatus to execute the steps of:
generating a machine learning model by using training data, the training data being skin class information that is generated by analyzing biological information of multiple users provided by the multiple users and that classifies skins of the multiple users, the machine learning model estimating a skin class of the user from at least one of skeletal feature information or skin color information obtained by analyzing face images of the multiple users, the skeletal feature information indicating features of facial skeleton of the multiple users, the skin color information indicating features of skin colors of the multiple users;
receiving an input of a face image of a user;
analyzing the received face image to obtain at least one of the skeletal feature information or the skin color information; and
outputting skin class information estimated from at least one of the obtained skeletal feature information or skin color information by using the machine learning model.
